# EUROPEAN PATENT SPECIFICATION

(11) **EP 2 482 759 B1**
(45) Date of publication and mention of the grant of the patent: **20.04.2016**
(21) Application number: 10763593.0
(22) Date of filing: 30.09.2010
(51) Int. Cl.: A61F 2/00

(54) **BODY IMPLANTABLE FABRIC TO REPAIR A PELVIC FLOOR**
KÖRPERIMPLANTIERBARES GEWEBE ZUR REPARATUR EINES BECKENBODENS
TISSU IMPLANTABLE DANS LE CORPS POUR RÉPARER LE PLANCHER PELVIEN

(30) Priority: 30.09.2009 DK 200970135; 01.10.2009 US 571444
(43) Date of publication of application: 08.08.2012
(73) Proprietor: Coloplast A/S, 3050 Humlebæk (DK)
(72) Inventor: DEITCH, Sarah, J., Stillwater MN 55082 (US)
(86) International application number: PCT/DK2010/050248
(87) International publication number: WO 2011/038740

(56) References cited:
- WO-A1-00/07520
- WO-A1-2006/071023
- WO-A2-2009/005714
- US-A1- 2008 065 229

## Description

### Background

Pelvic organ prolapse refers to a disorder related to the dropping down (prolapse) of the bladder, rectum, or uterus caused by weakness or injury to ligaments, connective tissue, or muscles of the pelvis. The various forms of pelvic organ prolapse are categorized according to the organ affected. For example, a rectocele develops when the rectum drops down and protrudes into the back wall of the vagina. An enterocele develops when the small intestine and the lining of the abdomen bulge downward between the uterus and the rectum or, if the uterus has been removed, between the bladder and the rectum. A cystocele develops when the bladder drops down and protrudes into the front wall of the vagina. In prolapse of uterus, the uterus drops down into the vagina.

Surgical procedures have been developed to repair pelvic organ prolapse, for example through the use of support materials implanted within the pelvis to strengthen the ligaments, connective tissue, or muscles of the pelvis. While the surgical procedures are generally effective, the patient can at times experience dysparunea (pain or the sensation of pain during vaginal intercourse), which is thought to be related to the undesired wrinkling or gathering of the implanted support material in the pelvic region.

WO 2009/005714 discloses pelvic implants and methods of surgically placing pelvic implants. The implants optionally include the ability to engage a spreader tool for spreading the implant within the patient and leave a frame.

### Summary

One aspect provides an implantable fabric according to claim 1.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a top view of an implantable fabric including a bio-inert portion reinforced by a bio-absorbable portion according to one embodiment.
Figure 2 is a cross-sectional view of the implantable fabric illustrated in Figure 1.
Figure 3 is a perspective view of another embodiment of an implantable fabric including a bio-inert portion reinforced by a bio-absorbable portion.
Figure 4 is a side view of the implantable fabric illustrated in Figure 3.
Figure 5 is a top view of another embodiment of an implantable fabric including a bio-inert portion reinforced by a bio-absorbable portion.
Figure 6 is a cross-sectional view of the implantable fabric illustrated in Figure 5.
Figure 7 is a top view of another embodiment of an implantable fabric including a bio-inert portion reinforced by a bio-absorbable portion.
Figure 8 is a cross-sectional view of implantable fabric illustrated in Figure 7.
Figure 9 is a top view of another embodiment of an implantable fabric including a bio-inert portion reinforced by a bio-absorbable portion.
Figure 10 is a cross-sectional view of the implantable fabric illustrated in Figure 9.
Figured 11A-11D are schematic illustrations of the implantable fabric illustrated in Figure 9 prepared for implantation and subsequently implanted into a patient's body according to one embodiment.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Tissue includes soft tissue, which includes dermal tissue, sub-dermal tissue, ligaments, tendons, or membranes. As employed in this specification, the term "tissue" does not include bone.

It is desirable to implant materials to support to the ligaments, connective tissue, or muscles of the pelvis in treating pelvic organ prolapse. However, some implanted materials have the potential to wrinkle or gather into folds, which is thought to undesirably contribute to dysparunea. Some implanted materials are configured to "self expand" after implantation, and the expansion has the potential to develop undesirable wrinkles/folds. Still other implanted materials have a "tissue adhesion barrier" side that prevents tissue ingrowth into both major surfaces of the implant.

Embodiments provide an implantable fabric suited for repair of pelvic organ prolapse, where the fabric is configured to allow tissue ingrowth through all of the major surfaces in a manner that ensures the implanted fabric will be strong enough to support the ligaments, connective tissue, or muscles of the pelvis in treating pelvic organ prolapse. The implantable fabric described herein is provided in a format that is compatible with conventional surgical implantation techniques and also prevents wrinkles on the implanted fabric, which helps to reduce or eliminate the undesirable effects of dysparunea.

Embodiments provide an implantable fabric suited for repair of pelvic organ prolapse that includes a bio-inert portion supported by a bio-absorbable portion. The bio-absorbable portion prevents wrinkles in the bio-inert portion as the bio-inert portion heals into place after implantation into the patient. Eventually, the bio-absorbable portion is bio-absorbed into the patient leaving behind a substantially wrinkle free bio-inert portion implanted within a patient to strengthen the ligaments, connective tissue, or muscles of the pelvis. The bio-inert portion is configured to allow tissue to grow through the top surface and the bottom surface of the bio-inert portion to effectively "anchor" the bio-inert portion within a patient.

Bio-inert means that the material is stable when implanted in an animal body.

Bio-absorbable means that the material will degrade or breakdown when implanted in an animal body. Bio-absorbable includes the disintegration of body implanted material, and the disintegration of body implanted material followed by the disintegrated material being absorbed into the body.

A wrinkle in the implantable fabric is an undulation in the fabric (e.g., a wavy fabric), where the undulation has more than one peak or more than one valley or where the fabric has portions that overlap. One particularly undesirable wrinkle is where the fabric has areas that overlap. Embodiments provide a fabric having bio-absorbable portion attached to at least one of the first and second surfaces of a bio-inert portion to support both of the first and second surfaces in a manner that substantially prevents the formation of a wrinkle on the surfaces after body implantation of the fabric.

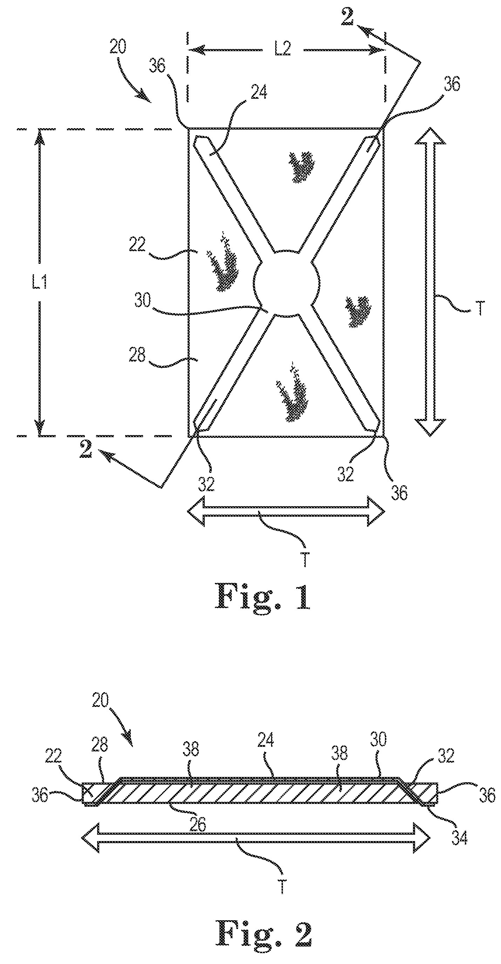

Figure 1 is a top view and Figure 2 is a cross-sectional view of one embodiment of an implantable fabric 20 suitable for use in the treatment of pelvic organ prolapse. In one embodiment, fabric 20 includes a bio-inert portion 22 that is reinforced by a bio-absorbable portion 24. For example, the bio-inert portion 22 includes a first major surface 26 (a bottom surface) opposite a second major surface 28 (a top surface) and the bio-absorbable portion 24 is mechanically attached to the bio-inert portion 22 to hold the bio-inert portion 22 under tension T in a manner that prevents wrinkles on the surfaces 26, 28.

In one embodiment, the bio-absorbable portion 24 prevents wrinkles along at least one major direction of the bio-inert portion 22. For example, in one embodiment the bio-absorbable portion 24 prevents wrinkles in the longitudinal direction by separating ends of the bio-inert portion 22, or the bio-absorbable portion 24 prevents wrinkles in the lateral direction by separating sides of the bio-inert portion 22. In one embodiment, the bio-absorbable portion 24 prevents wrinkles in both the longitudinal and lateral directions of the bio-inert portion 22.

In one embodiment, the bio-absorbable portion 24 includes a body 30, a flange 32 extending from the body 30, and a prong 34 extending from the flange 32 that is configured to mechanically engage with the bio-inert portion 22. In this embodiment, the bio-absorbable portion 24 is provided as a separate film that is fabricated to have a stiffness that is greater than a stiffness of the bio-inert portion 22. The flanges 32 and the prongs 34 combine to hold corners 36 of the fabric 20 apart under tension from one another when the bio-absorbable portion 24 is coupled to the bio-inert portion 22. Thus, the bio-absorbable portion 24 provides an extension member 24 that is mechanically coupled to one of the opposed major surfaces 26, 28 of the bio-inert portion 22.

The bio-inert portion 22 is selected to be bio-compatible with implantation into a human body and is configured to allow tissue ingrowth throughout its structure to anchor the bio-inert portion 22 in the body after implantation and healing. Suitable bio-inert portions 22 include autograft material (the patient's own tissue), allograft material (tissue from a cadaver), xenograft material (tissue from another species), or synthetic materials such as woven fabrics, meshes, nonwoven fabrics, meshes, fibrillated fibers, or spun and fibrillated fibers that are provided with voids (pores) configured to allow tissue ingrowth into the bio-inert portion 22. The pores are generally larger, on average, than 75 µm.

In one embodiment, the bio-inert portion 22 is a knitted monofilament polypropylene mesh provided as an approximately 225 cm² mesh having a weight of approximately 21 g/m² with a pore size of approximately 1121 µm and a thickness of approximately 260 µm. This mesh is thin and light weight (i.e., the basis weight is less than approximately 30 g/m²) to provide a thin and comfortable mesh that is less likely to erode tissue that contacts the mesh and less likely to be sensed through the tissue layers by the patient. Other suitable materials for bio-inert portion 22 include fabrics formed from polyester, polyethylene, silicone, urethanes, polyurethanes, copolymers, or block copolymers of these or suitably similar polymeric materials. Suitable such knitted monofilament polypropylene mesh is available from Coloplast Corp., Minneapolis, MN. Other suitable woven polypropylene mesh material is available from, for example, HerniaMesh, Chivasso, Italy.

The bio-absorbable portion 24 is attached to the bio-inert portion 22 and includes films that are pre-formed and subsequently attached to the bio-inert portion 22, films that are coated onto or into the bio-inert portion 22, or materials that are molded onto or into the bio-inert portion 22 that are subsequently cured or hardened to support the bio-inert portion 22. In one embodiment, the bio-absorbable portion 24 is formed as a semi-rigid plastic support that is mechanically fixed to the bio-inert portion 22 by the prongs 34. It is desirable that the bio-absorbable portion 24, when formed as a semi-rigid plastic support, be provided with less flexibility than the bio-inert portion 22 to allow the bio-absorbable portion 24 to tension the bio-inert portion 22 and prevents wrinkles on the surfaces 26, 28 of the bio-inert portion 22.

Suitable materials for the bio-absorbable portion 24 include biodegradable polymers, for example, polylactide, polyglycolide, poly(lactide-co-glycolide), or polylactic acid-based polymers. As noted, these bio-absorbable materials are suitably formed into film substrates, coatings, or injection molded supports for the bio-inert portion 22.

In one embodiment, bio-absorbable portion 24 is a molded semi-rigid film superstrate that is disposed across a portion of top surface 28 and coupled to a portion of bottom surface 26 by prongs 34 such that the bio-inert portion 22 is held in tension T. In particular, the prongs 34 displace the corners 36 apart from each other and hold the bio-inert portion 22 in tension T in a manner that prevents wrinkles on the surfaces 26, 28 of the bio-inert portion 22 until tissue grows through the surfaces 26, 28. In one embodiment, the bio-inert portion 22 includes voids 38 or pores 38 that are provided to facilitate tissue ingrowth through and into the bio-inert portion 22.

In one embodiment, the body 30 is provided as an X-shape configured to extend between the corners 36 and cover only a portion of one surface 26 or 28 of the bio-inert portion 22 (e.g., less than an entire surface 26 or 28 of the bio-inert portion 22).

In one embodiment, the bio-inert portion 22 is provided in rectangular form having sides that extend a length L1 between the opposing ends of the bio-inert portion 22, where the ends extent a length L2 between the opposing sides. The X-shape of the body 30 is sized to hold the ends of the bio-inert portion 22 apart by approximately the distance L1 and hold the sides apart by approximately the distance L2. In this manner, the bio-absorbable portion 24 supports the bio-inert portion 22 in a substantially wrinkle-free form that allows tissue ingrowth into the bio-inert portion 22. To this end, the bio-inert portion 22 heals into the implanted location without wrinkles or bulges, which reduces or eliminates the undesirable occurrence of dysparunea in the patient.

Either one or both of the bio-inert portion 22 and the bio-absorbable portion 24 may be coated or treated with an anti-microbial material, or a material that encourages/assists tissue ingrowth.

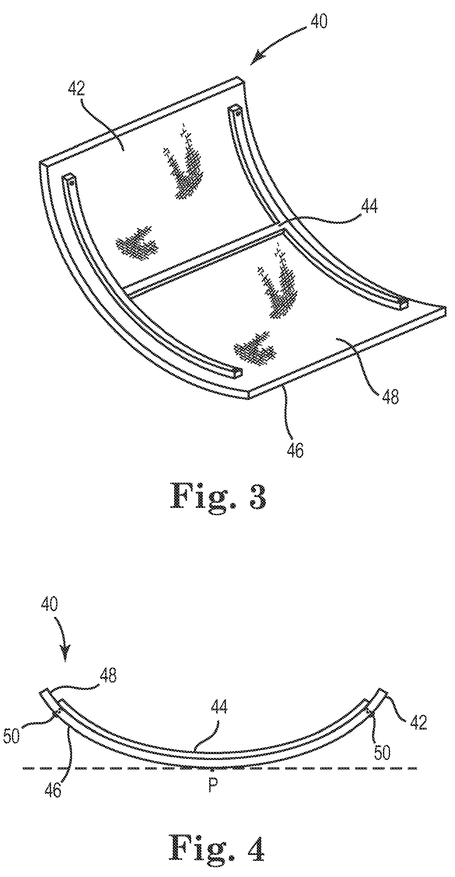

Figure 3 is a perspective view and Figure 4 is a side view of one embodiment of a body implantable fabric 40. In one embodiment, a body implantable fabric 40 includes a bio-inert portion 42 that is similar to the bio-inert portion 22 described above and a bio-absorbable portion 44 that is configured to support and prevents wrinkles in the bio-inert portion 42 until the body has an opportunity to heal and form tissue ingrowth through the bio-inert portion 42.

In one embodiment, the bio-inert portion 42 includes a first surface 46 opposite a second surface 48, and the bio-absorbable portion 44 is provided as an H-shaped semi-rigid support film that is tacked to the surface 48 by snaps 50. Snaps 50 extend from the first surface 46 through the bio-absorbable portion 44 and attach on the second surface 48. In one embodiment, the bio-absorbable portion 44 and the snaps 50 are both formed from bio-absorbable materials that are similar to those described above for the bio-absorbable portion 24.

In one embodiment, the bio-absorbable portion 44 maintains at least one of the first surface 46 or the second surface 48 of the bio-inert portion 42 in tension such that each of the first and second services 46, 48 are prevented from wrinkling and have at most one point of inflection P. Fabrics that are wrinkled will have more than one point of inflection (for example, at a bottom of each trough and at a top of each ridge). Implantable fabric 40 is supported by the bio-absorbable portion 44 and has at most one point of inflection P and is thus maintained in a substantially wrinkle-free conformation. One of ordinary skill in the art will recognize that a point of inflection is a point along a surface (for example a curved surface) where the slope of a line that is tangent to the surface changes sign.

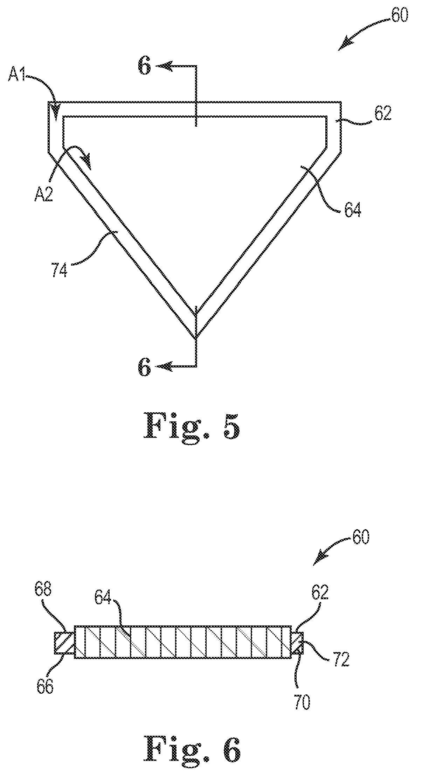

Figure 5 is a top view and Figure 6 is a cross-sectional view of one embodiment of a body implantable fabric 60. In one embodiment, the fabric 60 includes a bio-inert portion 62 supported by a bio-absorbable portion 64. The bio-inert portion 62 includes a first surface 66 opposite a second surface 68, and the bio-absorbable portion 64 is molded or injected or otherwise distributed through the bio-inert portion 62 between the surfaces 66, 68.

In one embodiment, bio-inert portion 62 is provided as an open fabric formed from fibers 70 that are separated by void areas 72, and the bio-absorbable portion 64 is molded through the bio-inert portion 62 around the fibers 70 and into the void areas 72. For example, in one embodiment the bio-inert portion 62 is provided as a mesh of fibers 70 having pores 72 associated with a pore size, and the bio-absorbable portion 64 fills at least some of the pores 72.

In one embodiment, the bio-inert portion 62 has an area A1 and the bio-absorbable portion 64 has an area A2, where the area A2 is less than the area A1. In this regard, the area A1 is larger than the area A2 such that a perimeter 74 of the bio-inert portion 62 is exposed and available for tissue ingrowth after body implantation of fabric 60. Thus, in this embodiment the area of bio-inert portion 62 is larger than the area of the bio-absorbable portion 64. The bio-absorbable portion 64 holds the bio-inert portion 62 under tension in a manner that prevents wrinkles on the surfaces 66, 68 of the bio-inert portion 62 until tissue grows through the surfaces 66, 68.

Suitable processes for attaching the bio-absorbable portion 64 to bio-inert portion 62 include polymer molding, injection molding, transfer molding, or pressing the bio-inert portion 62 between two sandwich layers of the bio-absorbable portion 64. As an example, in one embodiment the bio-inert portion 62 is placed into a mold and the bio-absorbable portion 64 is molded onto the surfaces 66, 68 and through the bio-inert portion 62. Implantation of the fabric 60 into the patient's body results in the bio-absorbable portion 64 eventually breaking down and being absorbed into the body, thus leaving the bio-inert portion 62 in place and substantially wrinkle-free to support tissues of the pelvis without the undesirable effects of dysparunea.

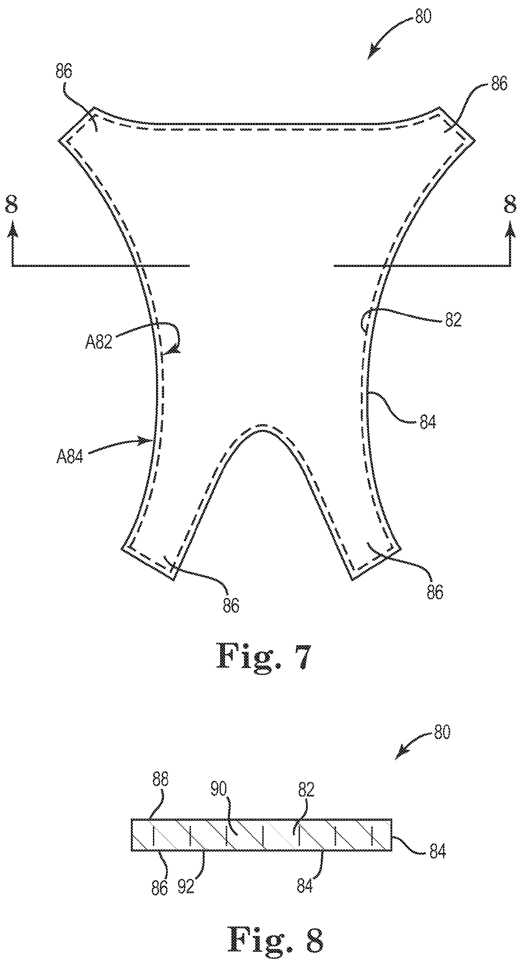

Figure 7 is a top view and Figure 8 is a cross-sectional view of one embodiment of a body implantable fabric 80. The fabric 80 includes a bio-inert portion 82 supported and surrounded by a bio-absorbable portion 84. In one embodiment, the bio-inert portion 82 includes a first major surface 86 opposite a second major surface 88, and the bio-absorbable portion 84 is molded through and around the bio-inert portion 82. For example, in one embodiment the bio-inert portion 82 is provided from one of the materials described above for bio-inert portion 22 and includes fibers 90 that are separated by voids 92, and the bio-absorbable portion 84 is molded around the fibers 90 and into substantially all of the voids 92.

In one embodiment, a bio-inert portion 82 is provided as a polygon including arms 86, and the bio-absorbable portion 84 is attached to the polygon and each of the arms 86. In this manner, the bio-inert portion 82 and the arms 86 are extended under tension in a manner that prevents wrinkles on the surfaces 86, 88. In one embodiment, the bio-inert portion 82 has an area A82 and the bio-absorbable portion 84 has an area A84, where the area A84 is greater than the area A82 (i.e., the bio-absorbable portion 84 is molded over the entire bio-inert portion 82).

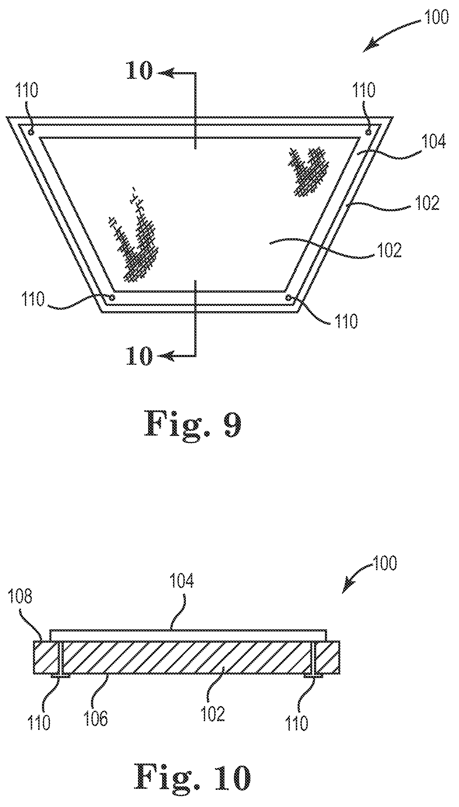

Figure 9 is a top view and Figure 10 is a cross-sectional view of one embodiment of a body implantable fabric 100. Body implantable fabric 100 includes a bio-inert portion 102 and a bio-absorbable portion 104. In one embodiment, the bio-inert portion 102 includes a first major surface 106 opposite a second major surface 108, and the bio-absorbable portion 104 is provided as a frame that is secured to a periphery of one of the surfaces 106, 108 of the bio-inert portion 102 by clips 110.

In one embodiment, the bio-absorbable frame 104 is integrated into the bio-inert portion 102 during fabrication of the fabric 100. In one embodiment, the bio-absorbable frame 104 is provided separately from the bio-inert portion 102 and is configured to be attached to the bio-inert portion 102 by a healthcare, for example, prior to body implantation.

In one embodiment, the bio-inert portion 102 is selected from one of the materials described above for the bio-inert portion 22 and the bio-absorbable portion 104 is molded as a semi-rigid frame from one of the materials described above for the bio-absorbable portion 24. The bio-absorbable frame 104 is attached to the periphery of the bio-inert portion 102 by the through-clips 110. It is desirable that the bio-absorbable portion 104 is less flexible than the bio-inert portion 102, and is yet configured to support the bio-inert portion 102 while allowing the bio-inert portion 102 to be rolled or folded or manipulated for implantation into the patient's body. Subsequent to implantation, the bio-absorbable portion 104 is broken down by the body to facilitate tissue ingrowth through the bio-inert portion 102.

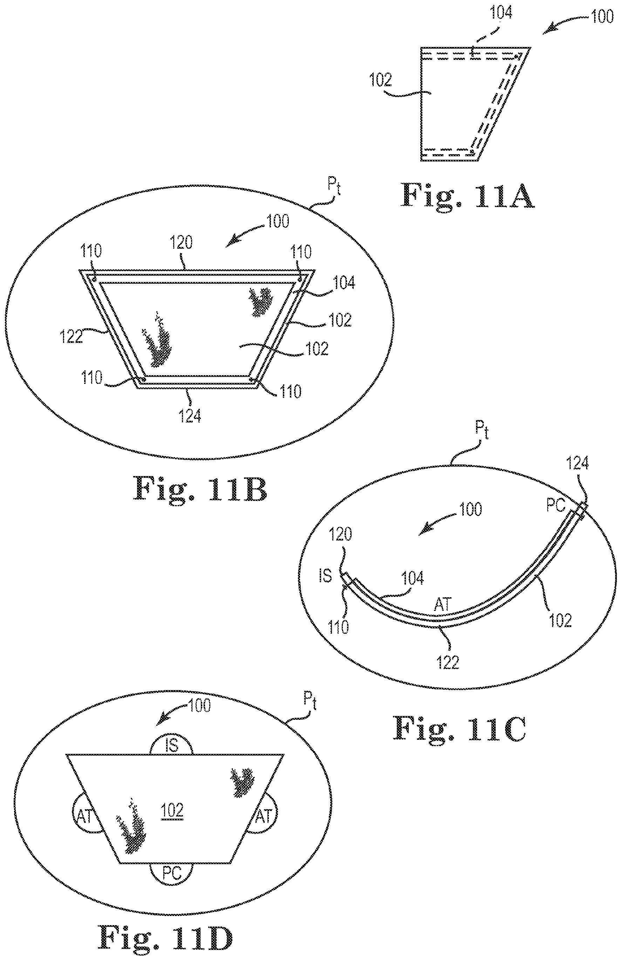

Figured 11A-11D are schematic illustrations of the implantable fabric 100 prepared for implantation (Figure 11A) and subsequently implanted into a patient's body Pt (Figures 11B-11D) according to one embodiment.

Figure 11A is a top view of the implantable fabric 100 folded in half to facilitate implantation into a patient. The bio-inert portion 102 has been folded to enclose the bio-absorbable portion 104 between layers of the bio entered portion 102. It is to be understood that the implantable fabric 100 could be rolled or folded multiple times to facilitate implantation into the patient Pt.

Figure 11B is a top schematic view and Figure 11C is a side schematic view of the body implantable fabric 100 implanted into the patient's body Pt. The bio-absorbable portion 104 maintains the bio-inert portion 102 in tension to prevent wrinkles in the top and bottom surfaces of the bio-inert portion 102. The bio-absorbable portion 104 eventually breaks down inside of the patient Pt, leaving the substantially wrinkle-free bio-inert portion 102 implanted inside the patient Pt to support the pelvic tissues.

In one exemplary approach, the top end 120 of the fabric 100 is attached to the ischial spine IS, a mid-portion 122 of the fabric 100 is attached to the arcus tendineus AT ligament near the pelvic fascia, and a lower end 124 of the fabric 100 is attached to the pubocervical PC connective tissue. The fabric 100 is thus suspended within the pelvis across the IS, AT, and PC tissues and the frame of the bio-absorbable portion 104 supports and maintains the bio-inert portion 102 to avoid the formation of wrinkles or overlapping areas of the bio-inert portion 102 until tissue ingrowth occurs.

Figure 11D is a top schematic view of the fabric 100 subsequent to implantation and after healing. The bio-absorbable portion 104 (Figure 11B) has been broken down by the patient's body Pt leaving the bio-inert portion 102 in place between the IS, AT, and PC tissues. As the bio-absorbable portion 104 is broken down by the body, the patient's tissue grows into the bio-inert portion 102. The bio-absorbable portion 104 prevents wrinkling of the bio-inert portion 102 until tissue ingrowth can occur to fully anchor the fabric 100 into the patient Pt.

With reference to Figures 11A-11D, one exemplary method of implanting a fabric into a patient's Pt body to repair pelvic organ prolapse includes attaching a bio-absorbable support 104 to the fabric 102, and tensioning the fabric 102 with the bio-absorbable support 104. The bio-absorbable support 104 is attached to the fabric 102 mechanically by snaps or prongs, or by other approaches, such as molding the support 104 over all or some of the fabric 102. Thereafter, the bio-absorbable support 104 and the fabric 102 are implanted into the body Pt and tissue is allowed to grow into opposed major surfaces (106, 108 in Figure 10) of the fabric 102 to provide a substantially non-wrinkled fabric 102 implanted in the body 104.

A body implantable fabric has been described having a light weight and flexible bio-inert portion that is supported by a bio-absorbable portion, where the bio-absorbable portion prevents wrinkles in the bio-inert portion until tissue ingrowth can occur through the bio-inert portion to anchor it within the patient's body.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention be limited only by the claims and the equivalents thereof.

## Claims

1. An implantable fabric (20) comprising:
a bio-inert portion (22) having a first surface (26) and an opposing second surface (28), each of the first and second surfaces configured for tissue ingrowth; and
a bio-absorbable portion (24) attached to the bio-inert portion (22) in a manner that substantially prevents wrinkles on each of the first and second surfaces (26, 28) of the bio-inert portion (22);
wherein the bio-absorbable portion (24) includes a body (30) provided as a separate film having a stiffness that is greater than a stiffness of the bio-inert portion (22), a flange (32) extending from the body (30), and a prong (34) extending from the flange (32) configured to mechanically engage with the bio-inert portion (22) through the bio-inert portion to hold corners (36) of the fabric (20) apart under tension from one another.

2. The implantable fabric (20) of claim 1, wherein the bio-inert portion (22) has first and second sides that extend a side length between first and second ends, and the bio-absorbable portion (24) stretches the first end apart from the second end by a distance of approximately the side length.

3. The implantable fabric (20) of claim 2, wherein the first and second ends each extend an end length between the first and second sides, and the bio-absorbable portion (24) stretches the first side apart from the second side by a distance of approximately the end length.

4. The implantable fabric (40) of claim 1, 2 or 3, wherein the bio-absorbable portion (44) maintains at least one of the first and second surfaces (46, 48) of the bio-inert portion (42) in tension such that each of the first and second surfaces (46, 48) have at most one point of inflection after implantation of the bio-inert portion.

5. The implantable fabric of any one of the claims 1 - 4, wherein the bio-inert portion (22) defines an area and the bio-absorbable portion (24) is attached to less than an entirety of the area of the bio-inert portion (22).

6. The implantable fabric of any one of the claims 1 - 5, wherein the bio-inert portion (62) defines an area and the bio-absorbable portion (64) is attached to an entirety of the area of the bio-inert portion.

7. The implantable fabric of any one of the claims 1 - 6, wherein the bio-absorbable portion (64) comprises a polymer that is molded into the bio-inert portion (62) between the first and second surfaces (66, 68).

8. The implantable fabric of claim 7, wherein the bio-inert portion (62) comprises a mesh having strands of fibers (70) separated by void areas (72) with a portion of the void areas (72) filled with the polymer of the bio-absorbable portion (64).

9. The implantable fabric of claim 7, wherein the bio-inert portion (82) comprises a mesh having strands of fibers separated by void areas (92) with an entirety of the void areas (92) filled with the polymer of the bio-absorbable portion (84).

10. The implantable fabric of any one of the claims 1 - 9, wherein the bio-absorbable portion (104) comprises a film that is mechanically attached to one of the first and second surfaces (106, 108) of the bio-inert portion (102).

11. The implantable fabric of any one of the claims 1 - 10, wherein the bio-inert portion (82) comprises a polygon and arms (86) extending from the polygon, and the bio-absorbable portion (84) is attached to the polygon and each of the arms (86) extending from the polygon.

## Patentansprüche

1. Implantierbares Gewebe (20), das Folgendes aufweist:
einen bioinerten Abschnitt (22), der eine erste Oberfläche (26) und eine gegenüberliegende zweite Oberfläche (28) aufweist, wobei jede der ersten und zweiten Oberfläche zum Gewebeeinwachsen konfiguriert ist; und
einen bioabsorbierbaren Abschnitt (24), der an dem bioinerten Abschnitt (22) in einer Weise befestigt ist, die im Wesentlichen Falten auf jeder der ersten und zweiten Oberfläche (26, 28) des bioinerten Abschnitts (22) verhindert;
wobei der bioabsorbierbare Abschnitt (24) einen Körper (30), der als eine getrennte Folie vorgesehen ist, die eine Steifigkeit aufweist, die größer als eine Steifigkeit des bioinerten Abschnitts (22) ist, einen Flansch (32), der sich vom Körper (30) erstreckt, und einen Ausläufer (34) aufweist, der sich vom Flansch (32) erstreckt, der konfiguriert ist, mechanisch mit dem bioinerten Abschnitt (22) durch den bioinerten Abschnitt in Eingriff zu treten, um Ecken (36) des Gewebes (20) unter Spannung voneinander entfernt angeordnet zu halten.

2. Implantierbares Gewebe (20) nach Anspruch 1, wobei der bioinerte Abschnitt (22) eine erste und zweite Seite aufweist, die sich über eine Seitenlänge zwischen einem ersten und zweiten Ende erstrecken, und der bioabsorbierbare Abschnitt (24) das erste Ende um einen Abstand von annähernd der Seitenlänge vom zweiten Ende weg zieht.

3. Implantierbares Gewebe (20) nach Anspruch 2, wobei sich das erste und zweite Ende jeweils über eine Endlänge zwischen der ersten und zweiten Seite erstrecken, und der bioabsorbierbare Abschnitt (24) die erste Seite um einen Abstand von annähernd der Endlänge von der zweiten Seite weg zieht.

4. Implantierbares Gewebe (40) nach Anspruch 1, 2 oder 3, wobei der bioabsorbierbare Abschnitt (44) mindestens eine der ersten und zweiten Oberfläche (46, 48) des bioinerten Abschnitts (42) unter Spannung hält, so dass jede der ersten und zweiten Oberfläche (46, 48) nach der Implantation des bioinerten Abschnitts höchstens einen Knickpunkt aufweist.

5. Implantierbares Gewebe nach einem der Ansprüche 1 bis 4, wobei der bioinerte Abschnitt (22) eine Fläche definiert und der bioabsorbierbare Abschnitt (24) an weniger als der Gesamtheit der Fläche des bioinerten Abschnitts (22) befestigt ist.

6. Implantierbares Gewebe nach einem der Ansprüche 1 bis 5, wobei der bioinerte Abschnitt (62) eine Fläche definiert und der bioabsorbierbare Abschnitt (64) an einer Gesamtheit der Fläche des bioinerten Abschnitts befestigt ist.

7. Implantierbares Gewebe nach einem der Ansprüche 1 bis 6, wobei der bioabsorbierbare Abschnitt (64) ein Polymer aufweist, das zwischen der ersten und zweiten Oberfläche (66, 68) in den bioinerten Abschnitt (62) geformt ist.

8. Implantierbares Gewebe nach Anspruch 7, wobei der bioinerte Abschnitt (62) ein Netz aufweist, das Stränge von Fasern (70) aufweist, die durch leere Bereiche (72) getrennt sind, wobei ein Anteil der leeren Bereiche (72) mit dem Polymer des bioabsorbierbaren Abschnitts (64) gefüllt ist.

9. Implantierbares Gewebe nach Anspruch 7, wobei der bioinerte Abschnitt (82) ein Netz aufweist, das Stränge von Fasern aufweist, die durch leere Bereiche (92) getrennt sind, wobei eine Gesamtheit der leeren Bereiche (92) mit dem Polymer des bioabsorbierbaren Abschnitts (84) gefüllt ist.

10. Implantierbares Gewebe nach einem der Ansprüche 1 bis 9, wobei der bioabsorbierbare Abschnitt (104) eine Folie aufweist, die mechanisch an einer der ersten und zweiten Oberfläche (106, 108) des bioinerten Abschnitts (102) befestigt ist.

11. Implantierbares Gewebe nach einem der Ansprüche 1 bis 10, wobei der bioinerte Abschnitt (82) ein Polygon und Arme (86) aufweist, die sich vom Polygon erstrecken, und der bioabsorbierbare Abschnitt (84) am Polygon und jedem der Arme (86) befestigt ist, die sich vom Polygon erstrecken.

## Revendications

1. Tissu implantable (20) comprenant :
une partie bioinerte (22) comportant une première surface (26) et une deuxième surface opposée (28), chacune des première et deuxième surfaces étant configurée pour une colonisation par les tissus ; et
une partie biorésorbable (24) fixée à la partie bioinerte (22) de manière à prévenir sensiblement la formation de rides sur chacune des première et deuxième surfaces (26, 28) de la partie bioinerte (22) ;
dans lequel la partie biorésorbable (24) comprend un corps (30) présent sous la forme d'un film séparé ayant une rigidité qui est supérieure à une rigidité de la partie bioinerte (22), un rebord (32) s'étendant à partir du corps (30), et une languette (34) s'étendant à partir du rebord (32), configurée pour coopérer mécaniquement avec la partie bioinerte (22) en s'insérant dans la partie bioinerte (22) pour maintenir les coins (36) du tissu (20) sous tension en les séparant les uns des autres.

2. Tissu implantable (20) selon la revendication 1, dans lequel la partie bioinerte (22) comporte des premier et deuxième côtés qui s'étendent sur une longueur latérale entre des première et deuxième extrémités, et la partie biorésorbable (24) soumet la première extrémité à une traction en l'écartant de la deuxième extrémité d'une distance correspondant approximativement à la longueur latérale.

3. Tissu implantable (20) selon la revendication 2, dans lequel les première et deuxième extrémités s'étendent chacune sur une longueur terminale entre les premier et deuxième côtés, et la partie biorésorbable (24) soumet le premier côté à une traction en l'écartant du deuxième côté d'une distance correspondant approximativement à la longueur terminale.

4. Tissu implantable (40) selon la revendication 1, 2 ou 3, dans lequel la partie biorésorbable (44) maintient au moins l'une des première et deuxième surfaces (46, 48) de la partie bioinerte (42) sous tension de telle sorte que chacune des première et deuxième surfaces (46, 48) comporte au maximum un point d'inflexion après l'implantation de la partie bioinerte.

5. Tissu implantable selon l'une quelconque des revendications 1 à 4, dans lequel la partie bioinerte (22) délimite une zone et la partie biorésorbable (24) est fixée à moins d'une totalité de la zone délimitée par la partie bioinerte (22).

6. Tissu implantable selon l'une quelconque des revendications 1 à 5, dans lequel la partie bioinerte (62) délimite une zone et la partie biorésorbable (64) est fixée à une totalité de la zone délimitée par la partie bioinerte.

7. Tissu implantable selon l'une quelconque des revendications 1 à 6, dans lequel la partie biorésorbable (64) comprend un polymère qui est formé par moulage dans la partie bioinerte (62) entre les première et deuxième surfaces (66, 68).

8. Tissu implantable selon la revendication 7, dans lequel la partie bioinerte (62) comprend un filet comportant des brins de fibres (70) séparées par des zones de vide (72), une partie des zones de vide (72) étant remplie avec le polymère de la partie biorésorbable (64).

9. Tissu implantable selon la revendication 7, dans lequel la partie bioinerte (82) comprend un filet comportant des brins de fibres séparées par des zones de vide (92), une totalité des zones de vide (92) étant remplie avec le polymère de la partie biorésorbable (84).

10. Tissu implantable selon l'une quelconque des revendications 1 à 9, dans lequel la partie biorésorbable (104) comprend un film qui est mécaniquement fixé à l'une des première et deuxième surfaces (106, 108) de la partie bioinerte (102).

11. Tissu implantable selon l'une quelconque des revendications 1 à 10, dans lequel la partie bioinerte (82) comprend un polygone et des bras (86) s'étendant à partir du polygone et la partie biorésorbable (84) est fixée au polygone, chacun des bras (86) s'étendant à partir du polygone.
